# EUROPÄISCHE PATENTSCHRIFT

(11) **EP 3 018 254 B1**
(45) Veröffentlichungstag und Bekanntmachung des Hinweises auf die Patenterteilung: **14.08.2019**
(21) Anmeldenummer: 15191111.2
(22) Anmeldetag: 22.10.2015
(51) Int. Cl.: E01C 19/48, G01J 5/00, G01J 5/02, G01J 5/04, G01J 5/08, G01N 33/42

(54) **VORRICHTUNG ZUR BESTIMMUNG DER TEMPERATUR EINES DURCH EINE BAUMASCHINE IN EINER EINBAUBREITE AUFGEBRACHTEN STRASSENBAUMATERIALS**
DEVICE FOR DETERMINING THE TEMPERATURE OF A ROAD BUILDING MATERIAL APPLIED ON A WIDTH OF SPREAD BY A CONSTRUCTION MACHINE
DISPOSITIF PERMETTANT DE DÉTERMINER LA TEMPÉRATURE D'UN MATÉRIAU DE VOIRIE APPLIQUÉ SUR UNE LARGEUR DE POSE PAR UN ENGIN DE CONSTRUCTION

(30) Priorität: 06.11.2014 DE 102014222693
(43) Veröffentlichungstag der Anmeldung: 11.05.2016
(73) Patentinhaber: MOBA Mobile Automation AG, 65555 Limburg (DE)
(72) Erfinder: Schönbach, Torsten, 65555 Limburg (DE); Becher, Dominik, 65555 Limburg (DE); Watermann, Marcus, 65555 Limburg (DE); Grasso, Christian, 65555 Limburg (DE)
(74) Vertreter: Zimmermann, Tankred Klaus

(56) Entgegenhaltungen:
- DE-U1-202009 016 129
- DE-U1-202013 001 597

## Beschreibung

Die vorliegende Erfindung bezieht sich auf eine Vorrichtung zur Bestimmung der Temperatur eines durch eine Baumaschine in einer Einbaubreite neu aufgebrachten Straßenbaumaterials gemäß dem Oberbegriff des Patentanspruchs 1.

Bei Straßenbauvorhaben, beispielsweise beim Neubau einer Straße oder einer Erneuerung eines beschädigten Straßenbelages, ist die Qualität des neu aufgebrachten Straßenbaumaterials anhand von Kontrollprüfungen durch die ausführenden Unternehmen zu dokumentieren. Dazu zählt auch die Messung der Temperatur der Asphaltschicht unmittelbar nach dem Einbau durch den Straßenfertiger. Dabei wird die Temperatur des neu aufgebrachten Straßenbaumaterials über die gesamte Einbaubreite unmittelbar hinter der Asphaltbohle des Straßenfertigers gemessen.

Aus der WO 0070150 A1 ist ein Fahrbahntemperaturüberwachungssystem mit einem Temperatursensor bekannt. Der Temperatursensor kann dabei entweder eine Wärmebildkamera, ein Thermoscanner oder eine Wärmebildkamera, die im "line scan"-Modus arbeitet, sein. Der Temperatursensor ist am hinteren Ende eines Straßenfertigers angeordnet, sodass die gesamte Breite der neu gelegten Asphaltschicht gescannt wird. Die aufgenommenen Temperaturwerte können auf einer Anzeigevorrichtung grafisch angezeigt werden.

Nachteilig an einem derartigen Temperatursensor ist, dass eine Wärmebildkamera bzw. ein Thermoscanner in der Regel sehr teuer in der Anschaffung ist. Insbesondere für kleinere Bauunternehmen ist eine derartige Anschaffung aufgrund der hohen Kosten meist nicht zu realisieren. Weiterhin nachteilig ist, dass die Erfassungs- bzw. Öffnungswinkel einer Wärmebildkamera bzw. ein Thermoscanners sehr begrenzt sind, sodass bei Einbaubreiten im Bereich von 8 bis 12 Metern beispielsweise an der Wärmebildkamera ein entsprechend angepasstes Objektiv angebracht werden muss, um die gesamte Einbaubreite des neu aufgebrachten Straßenbaumaterials erfassen zu können. Dadurch steigen wiederum die Kosten für einen derartigen Temperatursensor weiter an. Alternativ dazu müsste sowohl die Wärmebildkamera als auch der Thermoscanner in einer entsprechend hohen Position, d. h. weit über 4 Meter oberhalb der Oberfläche des neu aufgebrachten Straßenbaumaterials am Stra-βenfertiger angebracht werden, um die gesamte Einbaubreite des neu aufgebrachten Straßenbaumaterials erfassen zu können. Dies ist jedoch insbesondere bei Durchfahrten unter Brücken nachteilig.

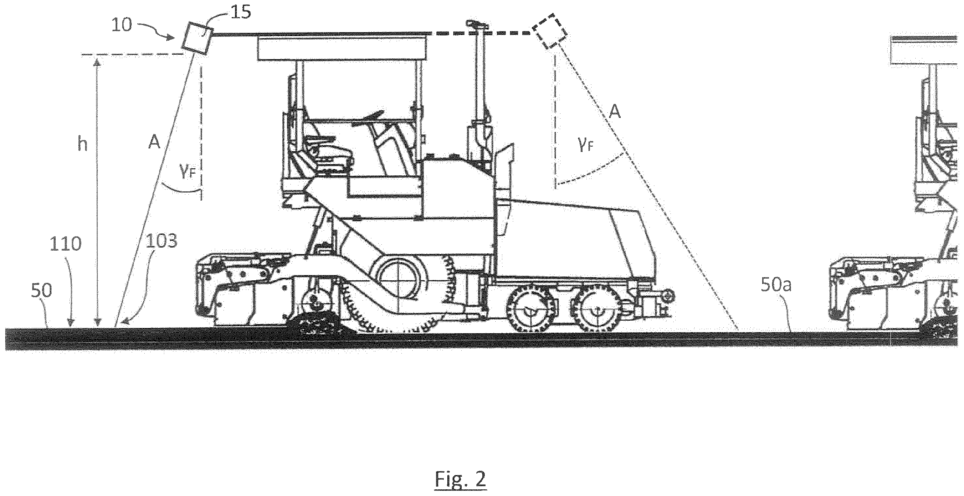

Wird jedoch die Wärmebildkamera bzw. der Thermoscanner im bevorzugten Bereich von 3 bis 4 Metern oberhalb der Oberfläche des neu aufgebrachten Straßenbaumaterials am Stra-βenfertiger montiert, so ist aufgrund des begrenzten Erfassungs- bzw. Öffnungswinkels ein entsprechend flacher Montagewinkel in Bezug zur Oberfläche des neu aufgebrachten Straßenbaumaterials erforderlich (vgl. hierzu Fig. 2, Montagewinkel γ_{F} muss groß sein), damit die gesamte Einbaubreite des neu aufgebrachten Straßenbaumaterials erfasst werden kann. Dadurch wird allerdings die Temperatur des neu aufgebrachten Straßenbaumaterials über die gesamte Einbaubreite nicht unmittelbar hinter der Asphaltbohle des Straßenfertigers gemessen, sondern in einer entsprechend großen Entfernung zur Hinterkante der Asphaltbohle. Die gemessenen Temperaturwerte entsprechen demnach nicht mehr den tatsächlichen Werten im Bereich unmittelbar hinter der Asphaltbohle.

Eine wie eingangs beschriebene Vorrichtung zur Temperaturmessung der Oberfläche von heißem Asphalt, bestehend aus einem sich quer zur Fahrtrichtung bewegenden Infrarottemperaturmesskopf, einem Motor zum Bewegen dieses Sensors und einer Ansteuerung, ist bereits aus der DE 20 2009 016 129 U1 bekannt.

Basierend auf dieser Vorrichtung ist aus der DE 20 2013 001 597 U1 eine Berechnung der Einbaubreite der neu aufgebrachten Asphaltschicht bekannt. Diese wird anhand der Höhe des Messkopfes über der Asphaltschicht, welche mit einem Abstandssensor ermittelt wird, und den Winkelwerten, an welchen der Messkopf die Bewegungsrichtung ändert, berechnet.

Mittels der bekannten Vorrichtung ergibt sich bei der Aufnahme der Temperaturmesswerte jedoch kein gleichbleibendes Messpunkteraster. Ändern sich die Montageposition und/oder die Montagewinkel der Vorrichtung, so ändert sich auch der Abstand der Messpunkte auf der Oberfläche des neu aufgebrachten Straßenbaumaterials. Weiterhin ändert sich der Abstand der Messpunkte in Fahrtrichtung der Baumaschine mit der Fahrgeschwindigkeit der Baumaschine. Fährt diese schneller, so wird der Abstand der Messpunkte in Fahrtrichtung größer.

Aufgabe der Erfindung ist es daher, eine einfache und kostengünstige Vorrichtung anzugeben, die es ermöglicht, Temperaturmesswerte von neu aufgebrachtem Straßenbaumaterial im Bereich unmittelbar hinter einer Baumaschine, insbesondere hinter einer Asphaltbohle eines Straßenfertigers, in einem gleichbleibenden Messpunkteraster über eine große Einbaubreite aufzunehmen.

Die Aufgabe wird gemäß den Merkmalen des unabhängigen Anspruches 1 gelöst. Bevorzugte Ausführungen der Erfindung sind Bestandteil von abhängigen Ansprüchen.

Gemäß Ausführungsbeispielen ist die Ansteuerung wirksam, den Motor ferner basierend auf den Montagewinkeln der Vorrichtung an der Baumaschine anzusteuern.

Dadurch wird auf der Oberfläche des neu aufgebrachten Straßenbaumaterials immer ein vorgegebener bzw. eingestellter Abstand von beispielsweise 25 cm zwischen zwei Messpunkten quer zur Fahrtrichtung der Baumaschine über die gesamte Einbaubreite beibehalten, unabhängig von der Montageposition und den Montagewinkeln der Vorrichtung. Ändern sich die Montageposition und/oder die Montagewinkel der Vorrichtung durch beispielsweise einen Umbau eines Werkzeuges an der Maschine, d. h. die Vorrichtung wird in der Höhe und/oder quer zur Fahrtrichtung der Baumaschine verschoben und/oder die Montagewinkel der Vorrichtung werden verändert, so wird der zuvor eingestellte Abstand von beispielsweise 25 cm zwischen zwei Messpunkten quer zur Fahrtrichtung der Baumaschine auch nach der Änderung der Montageposition und/oder der Montagewinkel über die Einbaubreite beibehalten.

Gemäß den Ausführungsbeispielen wird dies dadurch erreicht, dass bei einer sich ändernden Montageposition und/oder sich ändernden Montagewinkeln der Vorrichtung, die Motoransteuerung entsprechend angepasst und damit der vorgegebene bzw. eingestellte Abstand zwischen zwei Messpunkten quer zur Fahrtrichtung der Baumaschine wieder hergestellt und in vorteilhafter Weise nahezu konstant gehalten wird.

In vorteilhafter Weise kann der Infrarottemperaturmesskopf um einen sehr großen Winkel, beispielsweise im Bereich von ca. 120 ° bis 130 °, verdreht werden. Dadurch ist es mit der erfindungsgemäßen Vorrichtung möglich, Temperaturmesswerte in einem großen Bereich von bis zu 14 Meter Einbaubreite unmittelbar hinter einer Asphaltbohle eines Straßenfertigers bei einer bevorzugten Montagehöhe der Vorrichtung im Bereich von 3 bis 4 Metern oberhalb der Oberfläche des neu aufgebrachten Straßenbaumaterials zu erfassen. Die erfindungsgemäße Vorrichtung ist dabei nicht nur auf den Bereich großer Einbaubreiten beschränkt, sondern lässt sich aufgrund des variablen Verdrehwinkels des Infrarottemperaturmesskopfes für alle im zuvor genannten Bereich liegenden Einbaubreiten eines Straßenbelags verwenden. Gegenüber einer Wärmebildkamera bzw. einem Thermoscanner ist dies vorteilhaft, da diese üblicherweise einen festen Erfassungs- bzw. Öffnungswinkel aufweisen. Ferner stellen Durchfahrten des Straßenfertigers unter Brücken oder dergleichen kein Problem dar.

Weiterhin vorteilhaft sind die einfache Montage der Vorrichtung an der Baumaschine sowie die moderaten Kosten der einzelnen Komponenten der Vorrichtung und demgemäß die der gesamten Vorrichtung. Insbesondere ist ein Infrarottemperaturmesskopf um ein Vielfaches kostengünstiger gegenüber einer Wärmebildkamera oder einem Thermoscanner. Insofern ist die Anschaffung der erfindungsgemäßen Vorrichtung auch für kleinere Bauunternehmen realisierbar.

Mit der erfindungsgemäßen Vorrichtung ist es somit möglich, dass bei einer gleichbleibenden Einbaubreite des neu aufgebrachten Straßenbaumaterials eine immer gleichbleibende Anzahl von Messpunkten zur Ermittlung der Temperatur des neu aufgebrachten Straßenbaumaterials vorhanden ist.

Die Forderung nach einer gleichbleibenden Anzahl von Messpunkten bzw. einem gleichbleibenden Abstand zwischen zwei Messpunkten wird zukünftig Gegenstand von Ausschreibungen für Straßenbauvorhaben, beispielsweise für den Neubau einer Straße oder der Erneuerung eines beschädigten Straßenbelages, sein, um gleichbleibende und somit vergleichbare Qualitätsmessungen des neu aufgebrachten Straßenbaumaterials zu erhalten.

Gemäß Ausführungsbeispielen ändert sich die Bewegungsgeschwindigkeit des Infrarottemperaturmesskopfs in Abhängigkeit von der Fahrgeschwindigkeit der Baumaschine. Dadurch wird auf der Oberfläche des neu aufgebrachten Straßenbaumaterials ein immer gleicher Abstand der Messpunkte in Fahrtrichtung der Baumaschine, d. h. ein gleicher Abstand der Messreihen, unabhängig von der Fahrgeschwindigkeit der Baumaschine, erzielt. Soll beispielsweise der Abstand der Messreihen, d. h. der Messpunkte in Fahrtrichtung der Baumaschine, immer 25 cm betragen, so muss bei Erhöhung der Fahrgeschwindigkeit der Baumaschine ebenfalls die Bewegungsgeschwindigkeit des Infrarottemperaturmesskopfs erhöht werden und umgekehrt.

Um einen annähernd gleichbleibenden Abstand der Messreihen zu erhalten, ist die Ansteuerung für den Motor oder eine an der Vorrichtung oder an der Baumaschine angeordnete Auswerteeinheit in vorteilhafter Weise mit dem Baumaschinen-Steuerungsrechner oder einer an der Baumaschine angeordneten Wegmesseinrichtung, wie bspw. ein bei Straßenfertigern üblicherweise eingesetztes Wegrad, elektrisch verbunden. Der somit erhaltene Geschwindigkeitswert kann dann für eine Berechnung der Bewegungsgeschwindigkeit des Infrarottemperaturmesskopfs verwendet werden. Die Berechnung der Bewegungsgeschwindigkeit des Infrarottemperaturmesskopfs kann dabei entweder in der Ansteuerung für den Motor oder in der an der Vorrichtung oder an der Baumaschine angeordneten Auswerteeinheit erfolgen.

Der Vorteil der Anpassung der Bewegungsgeschwindigkeit des Infrarottemperaturmesskopfs an die Fahrgeschwindigkeit der Baumaschine liegt darin, dass in Verbindung mit einem gleichen Messpunktabstand quer zur Fahrtrichtung der Baumaschine, d. h. in Bewegungsrichtung des Infrarottemperaturmesskopfes, ein homogenes Netz aus Messpunkten entsteht. Es werden nur so viele Messpunkte vom Infrarottemperaturmesskopf aufgenommen, wie für die Darstellung und Protokollierung der gemessenen Temperaturmesswerte, beispielsweise auf einem Steuerungsrechner und/oder einer damit verbundenen Anzeige- und Bedieneinheit, benötigt werden. Ein Nachbearbeiten der aufgenommenen Temperaturmesswerte, beispielsweise durch den Steuerungsrechner, wie das Verwerfen bzw. Löschen von nicht benötigten Messwerten bzw. Messwertreihen oder eine Interpolation von Messwerten bzw. Messwertreihen, entfällt. Auch für eine Übertragung der Daten zu anderen Baumaschinen, wie beispielsweise eine Walze, um die Daten dem Walzenfahrer komprimiert und in einer einfachen Art auf einer Anzeigeeinheit darzustellen, ist eine auf ein Minimum begrenzte Menge der zu übertragenden Daten vorteilhaft.

Gegenüber einer Wärmebildkamera oder einem Thermoscanner ist dies vorteilhaft, da diese üblicherweise eine sehr hohe Auflösung aufweisen. Es werden in der Regel viel mehr Messpunkte aufgenommen, wie für die Darstellung und Protokollierung der gemessenen Temperaturmesswerte, beispielsweise auf einem Steuerungsrechner und/oder einer damit verbundenen Anzeige- und Bedieneinheit, benötigt werden. Dadurch entsteht gegebenenfalls eine hohe Menge an Daten, welche durch den Steuerungsrechner verarbeitet werden müssen.

Gemäß Ausführungsbeispielen wechselt die Bewegungsrichtung des Infrarottemperaturmesskopfs, sobald die gemessene Temperatur an mindestens einem Messpunkt einen vorbestimmten Mindestwert, z.B. 80 °C, unterschreitet. Der Infrarottemperaturmesskopf, welcher durch den Motor quer zur Fahrtrichtung der Baumaschine bewegt wird, misst kontinuierlich die Oberflächentemperatur des neu aufgebrachten Straßenbaumaterials. Die Temperaturwerte liegen üblicherweise im Bereich von 120 bis 170 °C. An Stellen, an denen Temperaturwerte im Bereich von 80 bis 120 °C gemessen werden, wurde zu kaltes Straßenbaumaterial eingebaut - es entsteht in der neu aufgebrachten Straßenschicht ein sogenannter "Cold Spot", was die Qualität des Straßenbelags mindert. Wird allerdings vom Infrarottemperaturmesskopf eine Temperatur von weniger als beispielsweise 80 °C gemessen, so ist davon auszugehen, dass eine der beiden Außenkanten, d. h. das seitliche Ende der neu aufgebrachten Straßenschicht, erreicht ist.

Dabei ist es auch möglich, dass ein sogenannter "Cold Spot" im Bereich unter dem vorbestimmten Mindestwert von bspw. 80 °C liegen kann. Um in diesem Fall eine vorzeitige und ggf. fehlerhafte Änderung der Bewegungsrichtung des Infrarottemperaturmesskopfes zu vermeiden, wird der Infrarottemperaturmesskopf zunächst bis zum Erreichen der zuvor ermittelten Außenkante bewegt und die aufgenommenen Temperaturmesswerte der derzeitig durchgeführten Messreihe mit den Werten mindestens einer der zuvor aufgenommenen Messreihen verglichen.

Wird dabei an den Messpunkten im Randbereich des Straßenbelags, d. h. im Bereich der Außenkanten, mindestens ein Temperaturwert ermittelt, welcher über dem vorbestimmten Mindestwert, d. h. oberhalb von bspw. 80 °C, liegt, dann ist davon auszugehen, dass sich die derzeitige Einbaubreite nicht verringert hat und ein sogenannter "Cold Spot" in der neu aufgebrachten Straßenschicht vorhanden ist. Der Infrarottemperaturmesskopf wird dann in vorteilhafter Weise bis zur zuvor ermittelten Außenkante oder auch darüber hinaus weiterbewegt, bis an mindestens einem Messpunkt eine Temperatur von weniger als beispielsweise 80 °C gemessen wird. Dann ist davon auszugehen, dass eine der beiden Außenkanten, d. h. das seitliche Ende der neu aufgebrachten Straßenschicht, erreicht ist.

Werden an den Messpunkten im Bereich der Außenkanten nur Temperaturwerte unter dem vorbestimmten Mindestwert, d. h. unterhalb von bspw. 80 °C, ermittelt, dann ist davon auszugehen, dass sich entweder die derzeitige Einbaubreite verringert hat oder sich ein sogenannter "Cold Spot" im Randbereich des Straßenbelags, d. h. im Bereich der Außenkanten, befindet. Der Infrarottemperaturmesskopf wird bei den darauffolgenden Messreihen in vorteilhafter Weise den Messbereich verringern und sich somit an eine geänderte Einbaubreite bzw. Außenkante annähern. Dazu wird der Infrarottemperaturmesskopf nur noch so weit verdreht werden, bis an mindestens einem Messpunkt eine Temperatur von weniger als beispielsweise 80 °C gemessen wird. Dann muss der Infrarottemperaturmesskopf davon ausgehen, dass eine der beiden Außenkanten, d. h. das seitliche Ende der neu aufgebrachten Straßenschicht, erreicht ist.

Bei einer Verringerung der Einbaubreite verringert sich aufgrund des gleichbleibenden Abstandes der Messpunkte folglich auch die Anzahl der Messpunkte, an welchen der Infrarottemperaturmesskopf Messwerte aufnimmt. Im umgekehrten Fall, d. h. bei einer Verbreiterung der Einbaubreite, erhöht sich demgemäß die Anzahl der Messpunkte.

Gemäß Ausführungsbeispielen wird die Position, an welcher der Infrarottemperaturmesskopf die Bewegungsrichtung wechselt, in der Ansteuerung oder einer an der Vorrichtung oder an der Baumaschine angeordneten Auswerteeinheit für eine Berechnung der Einbaubreite des neu aufgebrachten Straßenbaumaterials abgespeichert. Aus den abgespeicherten Winkelpositionen des Infrarottemperaturmesskopfs sowie der Höhe und den Montagewinkeln der Vorrichtung bzw. des Infrarottemperaturmesskopfs zur Oberfläche des neu aufgebrachten Straßenbaumaterials wird die Einbaubreite des neu aufgebrachten Straßenbaumaterials berechnet.

Erfindungsgemäß ist der Abstand der Messpunkte und/oder die Zeitdauer der Temperaturmessung an einem Messpunkt einstellbar. Dabei wird der Abstand zwischen zwei Messpunkten sowohl quer zur Fahrtrichtung der Baumaschine als auch in Fahrtrichtung der Baumaschine vorzugsweise durch Programmierung der Ansteuerung, beispielsweise über einen Steuerungsrechner oder einer damit verbundenen Anzeige- und Bedieneinheit, eingestellt. Ebenso kann die Zeitkonstante des Infrarottemperaturmesskopfs, d. h. die Zeitdauer der Temperaturmessung an einem Messpunkt, vorzugsweise durch Programmierung, beispielsweise über einen Steuerungsrechner oder einer damit verbundenen Anzeige- und Bedieneinheit, eingestellt werden. In vorteilhafter Weise ist es dadurch möglich, die Vorrichtung an die beispielsweise länderspezifischen Anforderungen anzupassen. Da in den USA eine neue Straßenschicht mit einer höheren Fahrgeschwindigkeit des Straßenfertigers eingebaut wird, muss die Zeitdauer der Temperaturmessung an einem Messpunkt dazu verkürzt werden. Auch beträgt in den USA der Abstand zwischen zwei Messpunkten in der Regel ca. 30 cm, wohingegen derzeit in Deutschland ein Messpunktabstand von ca. 25 cm forciert wird.

Gemäß Ausführungsbeispielen ist im Bereich der Vorrichtung ein berührungsloser Entfernungsmesser, beispielsweise ein Laserentfernungsmesser, angeordnet, mittels welchem der Abstand des Infrarottemperaturmesskopfes zum Messpunkt, an welchem der Infrarottemperaturmesskopf in Fahrtrichtung der Baumaschine im Wesentlichen senkrecht zur Oberfläche des Straßenbaumaterials angeordnet ist, gemessen wird. Gegenüber einer Höhenmessung durch einen der Maschinenbediener, beispielsweise mit einem Maßband, ist eine derartige Abstands- bzw. Entfernungsmessung vorteilhaft, denn der gemessene Wert kann vor Beginn der Bauarbeiten auf der Anzeige- und Bedieneinheit abgelesen werden und anschließend in die Ansteuerung der Vorrichtung programmiert werden. Zur Entfernungsmessung werden vorzugsweise Lasersensoren nach dem Prinzip der Lichtlaufzeitmessung verwendet, es können jedoch auch Ultraschallsensoren oder andere Sensortechnologien verwendet werden.

Gemäß Ausführungsbeispielen ist der berührungslose Entfernungsmesser ein Teil der erfindungsgemäßen Vorrichtung. Alternativ kann der berührungslose Entfernungsmesser ein externer Sensor sein, der z.B. an einer geeigneten Stelle an der Vorrichtung oder an der Baumaschine angeordnet ist und mit der Vorrichtung verbunden ist.

Gemäß Ausführungsbeispielen ist der berührungslose Entfernungsmesser mit der Ansteuerung der Vorrichtung elektrisch verbunden. Gegenüber einer manuellen Programmierung des Höhenwertes in die Ansteuerung, beispielsweise über einen Steuerungsrechner oder einer damit verbundenen Anzeige- und Bedieneinheit, ist es von Vorteil, wenn der gemessene Wert vom Entfernungsmesser zur Ansteuerung unmittelbar übertragen wird. Dadurch werden beispielsweise fehlerhafte Eingaben durch einen der Maschinenbediener vermieden.

Gemäß Ausführungsbeispielen ist die Ansteuerung mit einer an der Baumaschine angeordneten Wetterstation, welche beispielsweise Windgeschwindigkeit, Umgebungstemperatur, Luftfeuchte, Niederschlagsmenge und/oder andere Umgebungsparameter im Bereich der Baumaschine ermittelt, elektrisch verbunden. Die Wetterstation überträgt dabei die ermittelten Messwerte zur Ansteuerung, welche diese für weitere Berechnungen, wie beispielsweise eine Berechnung der Kerntemperatur des neu aufgebrachten Straßenbaumaterials, verwendet bzw. abspeichert.

Gemäß Ausführungsbeispielen ist der Motor ein Schrittmotor, ein Servomotor, ein Gleichstrommotor oder ein Gleichstrommotor mit Getriebe.

Die vorliegende Erfindung schafft auch eine Baumaschine, insbesondere ein Straßenfertiger, mit mindestens einer erfindungsgemäßen Vorrichtung, wobei die Vorrichtung im hinteren Bereich und/oder im vorderen Bereich der Baumaschine angeordnet ist.

Ausführungsbeispiele der vorliegenden Erfindung werden nachfolgend unter Bezugnahme auf die beiliegenden Zeichnungen näher erläutert. Es zeigen:
Fig. 1 einen schematischen Aufbau der erfindungsgemäßen Vorrichtung;
Fig. 2 eine schematische Darstellung eines Straßenfertigers mit jeweils einer erfindungsgemäßen Vorrichtung im vorderen sowie im hinteren Bereich;
Fig. 3 eine schematische Darstellung zur Erläuterung der Arbeitsweise der erfindungsgemäßen Vorrichtung;
Fig. 4 eine schematische Darstellung der in Fig. 3 dargestellten Arbeitsweise, jedoch mit einer in Bezug zur Fahrtrichtung der Baumaschine zur rechten Seite versetzt angeordneten Vorrichtung;
Fig. 5 eine schematische Darstellung der in Fig. 3 dargestellten Arbeitsweise, jedoch mit einer in Bezug zur Fahrtrichtung der Baumaschine zur linken Seite versetzt angeordneten Vorrichtung;
Fig. 6 eine schematische Darstellung gemäß Fig. 5, wobei die Vorrichtung um einen Montagewinkel entlang der Scanrichtung des Infrarottemperaturmesskopfes verdreht angeordnet ist;
Fig. 7 eine schematische Darstellung eines Straßenfertigers mit einer an dessen hinteren Ende angeordneten erfindungsgemäßen Vorrichtung; und
Fig. 8 eine schematische Darstellung des in Fig. 7 dargestellten Straßenfertigers mit einem auf der Oberfläche des neu aufgebrachten Straßenbaumaterials schematisch dargestellten Messpunkteraster.

In der nachfolgenden Beschreibung der Ausführungsbeispiele werden in den beiliegenden Zeichnungen gleiche oder gleichwirkende Elemente mit den gleichen Bezugszeichen versehen.

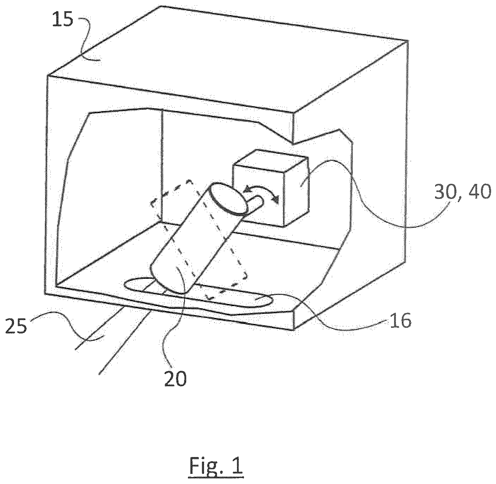

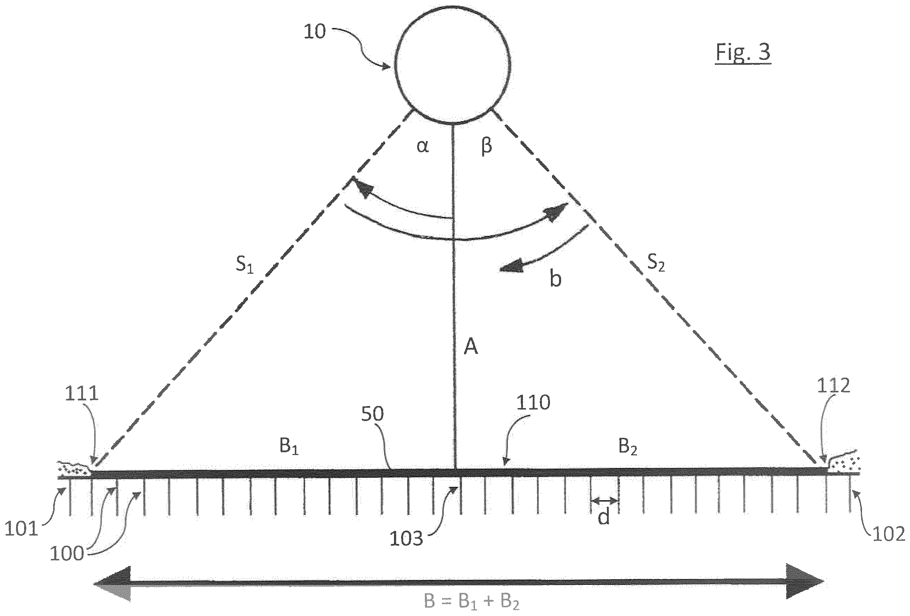

In Fig. 1 ist schematisch eine erfindungsgemäße Vorrichtung dargestellt, welche im Wesentlichen aus einem Motor 30, einem an dem Motor 30 bzw. an der Motorachse angeordneten Infrarottemperaturmesskopf 20 sowie einer im Bereich des Motors 30 angeordneten Ansteuerung 40 besteht. Alle genannten Komponenten sind geschützt in einem Gehäuse 15 angeordnet, wobei das Gehäuse 15 in seinem unteren Bereich, d. h. in Richtung der Oberfläche 110 eines neu aufgebrachten Straßenbelags (hier nicht dargestellt), eine im Wesentlichen längliche Öffnung 16 aufweist. Dadurch, dass der Infrarottemperaturmesskopf 20 am Motor 30 bzw. an der Motorachse angeordnet ist, wird bei einer Drehbewegung der Motorachse der Infrarottemperaturmesskopf 20 ebenfalls verdreht. Dies wird in der Figur schematisch durch eine gestrichelte Position des Infrarottemperaturmesskopfes 20 angedeutet. Vorzugsweise lässt sich der Infrarottemperaturmesskopf 20 in einem Winkelbereich von ca. 120 ° bis 130 ° verdrehen. Während einer Drehbewegung nimmt der Infrarottemperaturmesskopf 20 durch die Öffnung 16 an mindestens zwei voneinander beabstandeten Messpunkten 100 bis 103 auf der Oberfläche 110 des neu aufgebrachten Straßenbelags 50 (siehe z.B. Fig. 3) durch die von der Oberfläche 110 abgestrahlte Infrarotstrahlung 25 Temperaturmesswerte auf.

Fig. 2 zeigt die erfindungsgemäße Vorrichtung, welche an einer Position 10 in der Höhe h zur Oberfläche 110 des neu aufgebrachten Straßenbelags 50 im hinteren Bereich eines in seitlicher Ansicht dargestellten Straßenfertigers als auch in dessen vorderen Bereich (in der Figur gestrichelt dargestellt) angeordnet ist. Üblicherweise ist die Vorrichtung nur im hinteren Bereich des Straßenfertigers montiert und nimmt Temperaturmesswerte des neu aufgebrachten Straßenbelags 50 auf. Es ist jedoch auch denkbar, dass die Vorrichtung nur bzw. zusätzlich zu einer im hinteren Bereich angebrachten Vorrichtung auch im vorderen Bereich des Straßenfertigers, welcher beispielweise eine Asphalt-Deckschicht einbaut, angebracht ist. Bei einer Montage der Vorrichtung im vorderen Bereich des Straßenfertigers werden Temperaturmesswerte des zu asphaltierenden Untergrunds 50a aufgenommen, unabhängig davon, ob es sich dabei um einen durch einen weiteren Straßenfertiger zuvor eingebauten Straßenbelag, wie beispielsweise eine Asphalt-Binderschicht, handelt oder nicht.

Wie in Fig. 2 dargestellt, ist die Vorrichtung, unabhängig davon, ob diese im vorderen und/oder im hinteren Bereich des Straßenfertigers montiert ist, quer zur Fahrtrichtung des Straßenfertigers gesehen nicht senkrecht zur Oberfläche 110 am Straßenfertiger angeordnet, sondern in einem Montagewinkel γ_{F} im Bereich von z.B. 15 ° bis 30 ° in Bezug zur Senkrechten am Straßenfertiger montiert. Daraus folgt, dass der in den Figuren 2 bis 6 schematisch dargestellte Abstand A nicht zwingend gleich der Montagehöhe h der Vorrichtung(en) über der Oberfläche 110 sein muss, sondern dass der Abstand A immer der Abstand zwischen der Vorrichtung und dem Messpunkt 103 ist, an welchem der Infrarottemperaturmesskopf 20 in Fahrtrichtung der Baumaschine im Wesentlichen senkrecht zur Oberfläche 110 des Straßenbaumaterials 50 angeordnet ist. Um eine Messgenauigkeit von +/- 3 °C zu gewährleisten, ist es vorteilhaft, dass der Infrarottemperaturmesskopf 20 nicht über einen maximalen Montagewinkel γ_{F} von ca. 45 ° in Bezug zur Senkrechten am Straßenfertiger montiert wird.

Der in Fig. 3 dargestellte Infrarottemperaturmesskopf 20 bewegt sich in die durch das Bezugszeichen b bezeichneten Bewegungsrichtungen und nimmt während den Bewegungen Temperaturmesswerte an den im Abstand d dargestellten Messpunkten 100 bis 103 auf der Oberfläche 110 des neu aufgebrachten Straßenbelags 50 auf. Die Bewegungsrichtung des Infrarottemperaturmesskopfes 20 ändert sich, sobald die gemessene Temperatur an einem der Messpunkte 101 und 102 einen Mindestwert von z.B. 80 °C unterschreitet. Dazu werden die aufgenommenen Temperaturmesswerte der derzeitig durchgeführten Messreihe mit den Werten von mindestens einer der zuvor aufgenommenen Messreihen verglichen, um eine vorzeitige und ggf. fehlerhafte Änderung der Bewegungsrichtung des Infrarottemperaturmesskopfes 20 zu vermeiden. Die Messpunkte 101 und 102 liegen dabei außerhalb des Bereichs, in dem das Straßenbaumaterial 50 durch den Straßenfertiger aufgebracht wird. Dieser Bereich ist durch die beiden Außenkanten 111 und 112 in den Figuren 3 bis 8 angedeutet.

Alle Messpunkte 100 bis 103 sind in den Figuren 3 bis 6 schematisch als kurze, senkrechte und im Abstand d zueinander angeordnete Striche unterhalb des neu aufgebrachten Straßenbelags 50 markiert. Der Messpunkt 103 ist dabei der Messpunkt, an welchem der Infrarottemperaturmesskopf 20 in Fahrtrichtung der Baumaschine im Wesentlichen senkrecht zur Oberfläche 110 des Straßenbaumaterials 50 angeordnet ist. Ferner ist in den Figuren 3 bis 6 der Bereich, in welchem sich der Infrarottemperaturmesskopf 20 bewegt, durch die äußeren Infrarot-Strahlungslinien S₁ und S₂ angedeutet. Dadurch sind auch die beiden Winkel α und β definiert, welche jeweils von der Infrarot-Strahlungslinie S₁, S₂ bis zur Abstandslinie A reichen. Aus den beiden Winkelwerten α und β sowie aus dem bekannten Abstand A der Vorrichtung bzw. des Infrarottemperaturmesskopfes 20 zur Oberfläche 110 des neu aufgebrachten Straßenbelags 50 kann die Ansteuerung 40, welche den Motor 30 zum Verdrehen des Infrarottemperaturmesskopfes 20 ansteuert, oder auch eine außerhalb der Vorrichtung angeordnete Auswerte- bzw. Berechnungseinheit (hier nicht dargestellt), die beiden Teilabschnitte β₁ = tan α × A und B₂ = tan β × A berechnen. Die Gesamteinbaubreite B ergibt sich anschließend aus der Addition der beiden Teilabschnitte B₁ und B₂.

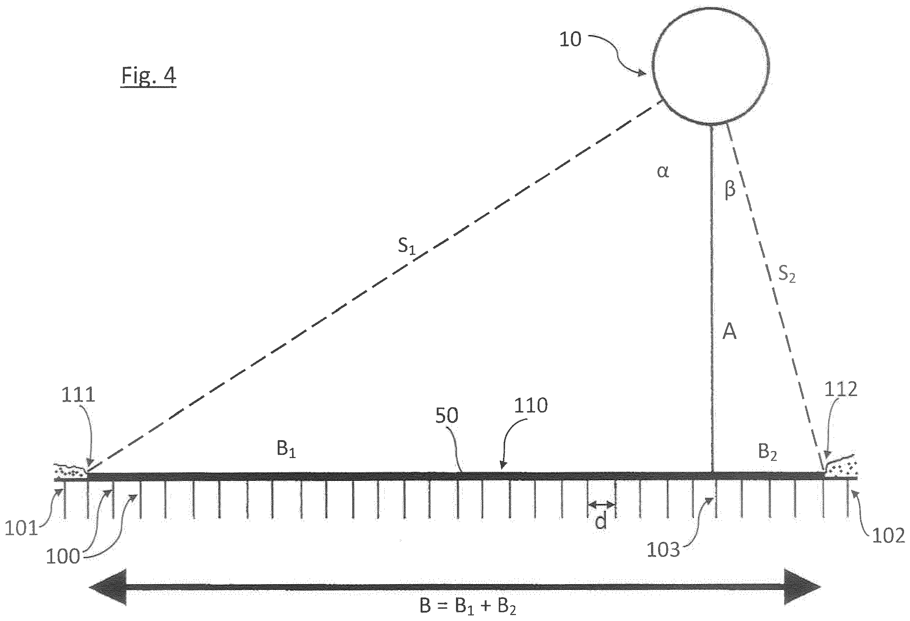

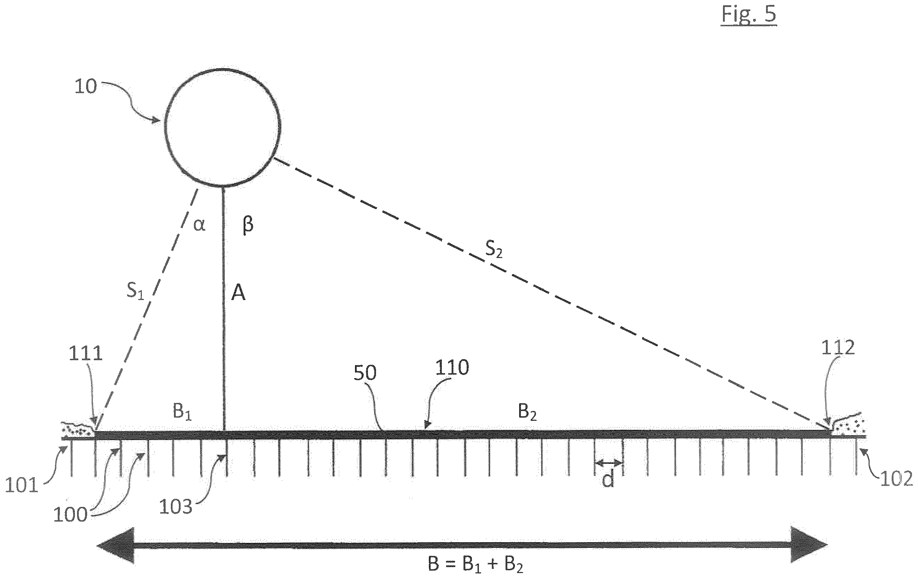

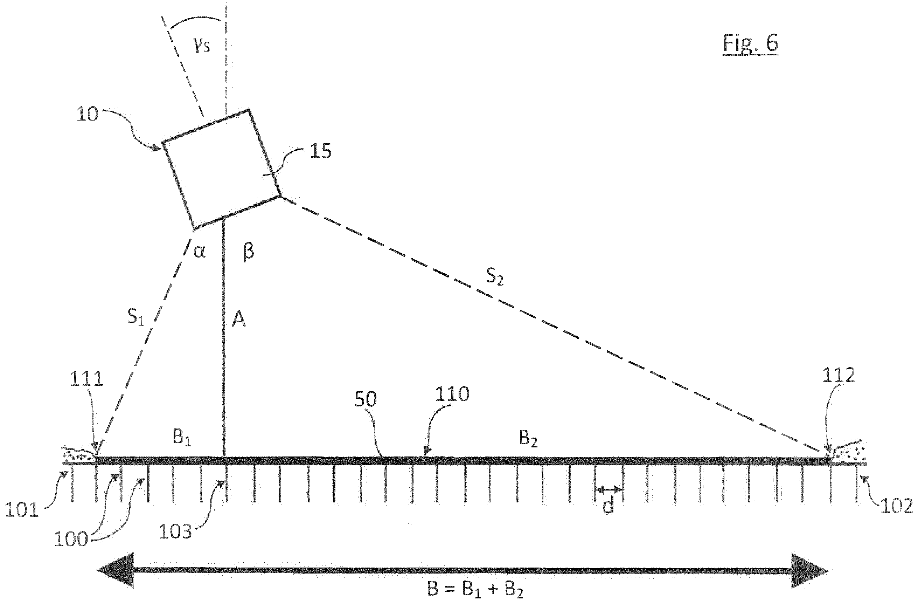

Ausgehend von Figur 3, in welcher die Vorrichtung quer zur Fahrtrichtung der Baumaschine (hier nicht dargestellt) im Wesentlichen mittig angeordnet ist, ist in den Figuren 4, 5 und 6 die Position 10 der Vorrichtung an dem Straßenfertiger (hier nicht dargestellt) geändert. D. h., in Fig. 4 ist die Vorrichtung in Bezug zur Fahrtrichtung des Straßenfertigers zur rechten Seite versetzt angeordnet, wohingegen in den Fig. 5 und 6 die Vorrichtung in Bezug zur Fahrtrichtung des Straßenfertigers zur linken Seite angeordnet ist und zusätzlich einen geringeren Abstand A in Bezug zur Oberfläche 110 des neu aufgebrachten Straßenbelags 50 aufweist. Bei den Beispielen in den Figuren 3 bis 6 wird der Einfachheit halber ein gleichbleibender Montagewinkel γ_{F} (vgl. Fig. 2) von beispielsweise 15 ° sowie ein Montagewinkel γ_{S} von 0 ° in Bezug zur Senkrechten vorausgesetzt. Der Abstand A berechnet sich somit aus A = (h / cos 15).

Im Beispiel gemäß Figur 3 wird demnach durch den Infrarottemperaturmesskopf 20 bei einem maximalen Gesamtverdrehwinkel α_{Max} + β_{Max} von beispielsweise ca. 120 ° und einer Montagehöhe h der Vorrichtung bzw. des Infrarottemperaturmesskopfes 20 eine Gesamtbreite von (tan α_{Max} × (h / cos 15)) + (tan β_{Max} × (h / cos 15)) = (tan 60 × (4 / cos 15)) + (tan 60 × (4 / cos 15)) ≈ 14,3 Meter erfasst. Bei einer angestrebten Erfassung eines neu aufgebrachten Straßenbelags 50 in einer Einbaubreite B von mindestens 8 Metern ist es von daher möglich, die Vorrichtung am Straßenfertiger quer zu dessen Fahrtrichtung um jeweils mehr als 3 m, ausgehend von der Mitte des Straßenfertigers, in Richtung der Außenkanten 111 und 112 zu verschieben, sodass der eingebaute Straßenbelag 50 in seiner gesamten Breite B immer noch erfasst wird.

In allen gemäß den Figuren 3 bis 6 dargestellten Ausführungsbeispielen wird somit immer die Oberfläche 110 des neu aufgebrachten Straßenbelags 50 über die gesamte Einbaubreite B vom Infrarottemperaturmesskopf 20 erfasst, auch wenn aufgrund des großen Verdrehwinkels des Infrarottemperaturmesskopfes 20 der Gesamterfassungsbereich wesentlich größer als die Einbaubreite B ist und die Vorrichtung quer zur Fahrtrichtung der Baumaschine gesehen nicht mittig angeordnet ist, wie dies in den Ausführungsbeispielen gemäß der Figuren 4 bis 6 der Fall ist.

Ferner ist in allen gemäß den Figuren 3 bis 6 dargestellten Ausführungsbeispielen die Ansteuerung 40 der Vorrichtung wirksam, den Motor 30 basierend auf der Montageposition 10 und den Montagewinkeln γ_{F} und γ_{S} der Vorrichtung an der Baumaschine derart anzusteuern, dass der Abstand d der Messpunkte 100 auf der zu messenden Oberfläche 110 gleich bleibt. Dies wird dadurch erreicht, dass die Ansteuerung 40 oder eine an der Vorrichtung oder an der Baumaschine angeordneten Auswerteeinheit (hier nicht dargestellt) den für den Infrarottemperaturmesskopf 20 einzustellenden Winkel α bzw. β zur Senkrechten, d. h. zum Messpunkt 103, an welchem der Infrarottemperaturmesskopf 20 in Fahrtrichtung der Baumaschine im Wesentlichen senkrecht zur Oberfläche 110 des Straßenbaumaterials 50 angeordnet ist, berechnet.

Im Beispiel der Ausführungsform gemäß Figur 3 sind die Winkel α und β gleich und betragen beispielsweise ca. 45 °. Ferner wird von einer Montagehöhe h der Vorrichtung bzw. des Infrarottemperaturmesskopfes 20 zur Oberfläche 110 des neu aufgebrachten Straßenbelags 50 von 4 Metern, einem einzustellenden Messpunkteabstand d von 25 cm sowie einer Ausgangsposition des Infrarottemperaturmesskopfes 20 in Richtung der Außenkante 111 ausgegangen. Demnach betragen die Abstände B₁ und B₂ jeweils B₁ = B₂ = (tan α × A) = tan α × (h / cos γ_{F}) = tan 45 × (4 / cos 15) = 3,86 Meter zu den Außenkanten 111 und 112.

Der erste aufzunehmende Messpunkt 100 im Bereich der Außenkante 111 erfolgt im Abstand von 3,75 m, ausgehend vom Messpunkt 103, welcher für den Infrarottemperaturmesskopf 20 eine sogenannte Nullposition bildet. Demnach wird für den Infrarottemperaturmesskopf 20 ein erster Verdrehwinkel α = arctan (3,75 / (h /cos γ_{F})) = arctan (3,75 / (4 / cos 15)) ≈ arctan 0,91 ≈ 42,16 ° eingestellt. Für den dann folgenden und im Abstand d = 0,25 m in Richtung der rechten Außenkante 112 liegenden Messpunkt 100 muss dann ein Verdrehwinkel von α = arctan ((3,75 - d) / (h /cos γ_{F})) = arctan ((3,75 - 0,25) / (4 / cos 15)) ≈ arctan 0,85 ≈ 40,20 ° eingestellt werden. Der darauf folgende Verdrehwinkel α beträgt dann arctan ((3,75 - 2d) / (h /cos γ_{F})) = arctan ((3,75 - 0,50) / (4 / cos 15)) = arctan 0,78 = 38,13 °. Die weiteren einzustellenden Verdrehwinkel α berechnen sich analog dazu.

In Annäherung an den Messpunkt 103, an welchem der Infrarottemperaturmesskopf 20 in Fahrtrichtung der Baumaschine im Wesentlichen senkrecht zur Oberfläche 110 des Straßenbaumaterials 50 angeordnet ist, beträgt der Verdrehwinkel α = arctan ((3,75 - 14d) / (h /cos γ_{F})) = arctan ((3,75 - 3,50) / (4 / cos 15)) ≈ arctan 0,06 ≈ 3,45 °. Beim Erreichen des Messpunktes 103 beträgt der einzustellende Verdrehwinkel α folglich 0 °, da der Infrarottemperaturmesskopf 20 sich dann wieder in der sogenannten Nullposition befindet. Die nun folgenden einzustellenden Verdrehwinkel berechnen sich analog der bisher durchgeführten Berechnungen und verwenden für die Berechnung den Winkel β. Demnach ist der erste, nach dem Messpunkt 103 folgende und in Richtung der rechten Außenkante 112 gerichtete, Verdrehwinkel β = arctan ((3,75 - 14d) / (h /cos γ_{F})) = arctan ((3,75 - 3,50) / (4 / cos 15)) ≈ arctan 0,06 ≈ 3,45 °. Für den dann folgenden und im Abstand d = 0,25 m in Richtung der rechten Außenkante 112 liegenden Messpunkt 100 muss dann ein Verdrehwinkel von β = arctan ((3,75 - 13d) / (h /cos γ_{F})) = arctan ((3,75 - 3,25) / (4 / cos 15)) ≈ arctan 0,12 ≈ 6,88 ° eingestellt werden. Der darauf folgende Verdrehwinkel β beträgt dann arctan ((3,75 - 12d) / (h /cos γ_{F})) = arctan ((3,75 - 3,00) / (4 / cos 15)) ≈ arctan 0,18 ≈ 10,27 °. Die weiteren einzustellenden Verdrehwinkel β des Infrarottemperaturmesskopfes 20 berechnen sich analog dazu.

In Fig. 4 ist aufgrund der versetzten Position 10 der Vorrichtung der Winkel α von ca. 60 ° wesentlich größer als der Winkel β. Der Infrarottemperaturmesskopf 20 kann jedoch um einen sehr großen Gesamtwinkel (α + β) von beispielsweise ca. 120 ° bis 130 ° verdreht werden. Um dabei jedoch eine Messgenauigkeit von +/- 3 °C zu gewährleisten, ist es vorteilhaft, dass der Infrarottemperaturmesskopf 20 nicht über einen maximalen Wert der beiden Winkeln α und β von jeweils ca. 60 ° in Bezug zur in Fahrtrichtung der Baumaschine senkrecht verlaufenden Abstandslinie A verdreht wird. Dennoch kann bei einer Positionierung 10 der Vorrichtung gemäß der Figur 4, d. h. beispielsweise bei einer Montagehöhe h der Vorrichtung im Bereich von ca. 4 Metern oberhalb der Oberfläche 110 des neu aufgebrachten Straßenbaumaterials 50 sowie einem seitlichen Abstand B₂ zur Außenkante 112 von ca. 1 Meter eine Gesamteinbaubreite B = B₁ + B₂ = (tan α × A) + 1 = (tan α × (h / cos 15)) + 1 = (tan 60 × (4 / cos 15)) + 1 ≈ 7,1 + 1 ≈ 8,1 Meter erfasst werden. Sofern die Vorrichtung in Bezug zur Fahrtrichtung des Straßenfertigers senkrecht zur Oberfläche 110 am Straßenfertiger angeordnet ist, d. h. der Montagewinkel γ_{F} = 0 ° in Bezug zur Oberfläche 110 beträgt, so ist der Abstand A von ca. 4 Metern im vorliegenden Beispiel gleich der Montagehöhe h der Vorrichtung über der Oberfläche 110.

Der am Beispiel gemäß Figur 4 erste aufzunehmende Messpunkt 100 im Bereich der Außenkante 111 erfolgt im Abstand von 7,00 m, ausgehend vom Messpunkt 103, welcher für den Infrarottemperaturmesskopf 20 die bereits genannte Nullposition bildet. Demnach wird für den Infrarottemperaturmesskopf 20 ein erster Verdrehwinkel α = arctan (7,00 / (h /cos γ_{F})) = arctan (7,00 / (4 / cos 15)) ≈ arctan 1,69 ≈ 59,4 ° eingestellt. Für den dann folgenden und im Abstand d = 0,25 m in Richtung der rechten Außenkante 112 liegenden Messpunkt 100 muss ein Verdrehwinkel von α = arctan ((7,00 - d) / (h /cos γ_{F})) = arctan ((7,00 - 0,25) / (4 / cos 15)) = arctan 1,63 ≈ 58,47 ° eingestellt werden. Der darauf folgende Verdrehwinkel α beträgt dann arctan ((7,00 - 2d) / (h /cos γ_{F})) = arctan ((7,00 - 0,50) / (4 / cos 15)) ≈ arctan 1,57 ≈ 57,5 °. Die weiteren einzustellenden Verdrehwinkel α berechnen sich analog dazu.

In Annäherung an den Messpunkt 103, an welchem der Infrarottemperaturmesskopf 20 in Fahrtrichtung der Baumaschine im Wesentlichen senkrecht zur Oberfläche 110 des Straßenbaumaterials 50 angeordnet ist, beträgt der Verdrehwinkel α = arctan ((7,00 - 27d) / (h /cos γ_{F})) = arctan ((7,00 - 6,75) / (4 / cos 15)) = arctan 0,06 ≈ 3,45 °, analog zum Beispiel der Ausführungsform gemäß Figur 3. Beim Erreichen des Messpunktes 103 beträgt der einzustellende Verdrehwinkel α folglich 0°, da der Infrarottemperaturmesskopf 20 sich dann wieder in der bereits genannten Nullposition befindet. Die nun folgenden einzustellenden Verdrehwinkel berechnen sich analog zum Beispiel der Ausführungsform gemäß Figur 3. Demnach ist der erste, nach dem Messpunkt 103 folgende und in Richtung der rechten Außenkante 112 gerichtete, Verdrehwinkel β ≈ 3,45 °. Für den dann folgenden und im Abstand d = 0,25 m in Richtung der rechten Außenkante 112 liegenden Messpunkt 100 muss dann ein Verdrehwinkel von β ≈ 6,88 ° eingestellt werden. Der darauf folgende Verdrehwinkel β beträgt dann ≈ 10,27 °. Die weiteren einzustellenden Verdrehwinkel β des Infrarottemperaturmesskopfes 20 berechnen sich analog dazu.

Im Gegensatz zu Fig. 4 ist in Fig. 5 der Winkel β von ca. 60 ° wesentlich größer als der Winkel α. Ferner ist die Montagehöhe h und damit auch der Abstand A der Vorrichtung bzw. des Infrarottemperaturmesskopfes 20 zur Oberfläche 110 des neu aufgebrachten Straßenbelags 50 geringer. Beispielsweise beträgt die Montagehöhe h ca. 3,5 m. Wird ein seitlicher Abstand B₁ zur Außenkante 111 von 2 Metern angenommen, so ist es auch bei dieser Positionierung 10 der Vorrichtung möglich, eine Gesamteinbaubreite B = B₁ + B₂ = 2 + (tan β × A) = 2 + (tan β × (h / cos 15)) = 2 + (tan 60 × (3,5 / cos 15)) ≈ 8,2 Meter zu erfassen. Auch bei diesem Beispiel ist der Abstand A von ca. 3,5 Metern gleich der Montagehöhe h der Vorrichtung über der Oberfläche 110, sofern die Vorrichtung in Bezug zur Fahrtrichtung des Straßenfertigers senkrecht zur Oberfläche 110 am Straßenfertiger angeordnet ist, d. h. der Montagewinkel γ_{F} = 0 ° in Bezug zur Oberfläche 110 beträgt.

Der am Beispiel gemäß Figur 5 erste aufzunehmende Messpunkt 100 im Bereich der Außenkante 111 erfolgt im Abstand B₁ von 2,00 m, ausgehend vom Messpunkt 103, welcher für den Infrarottemperaturmesskopf 20 die bereits genannte Nullposition bildet. Demnach wird für den Infrarottemperaturmesskopf 20 ein erster Verdrehwinkel α = arctan (2,00 / (h /cos γ_{F})) = arctan (2,00 / (3,5 / cos 15)) = arctan 0,55 ≈ 28,9 ° eingestellt. Für den dann folgenden und im Abstand d = 0,25 m in Richtung der rechten Außenkante 112 liegenden Messpunkt 100 muss ein Verdrehwinkel von α = arctan ((2,00 - d) / (h /cos γ_{F})) = arctan ((2,00 - 0,25) / (3,5 / cos 15)) ≈ arctan 0,48 ≈ 25,78 ° eingestellt werden. Der darauf folgende Verdrehwinkel α beträgt dann arctan ((2,00 - 2d) / (h /cos γ_{F})) = arctan ((2,00 - 0,50) / (3,5 / cos 15)) ≈ arctan 0,41 ≈ 22,49 °. Die weiteren einzustellenden Verdrehwinkel α berechnen sich analog dazu.

In Annäherung an den Messpunkt 103, an welchem der Infrarottemperaturmesskopf 20 in Fahrtrichtung der Baumaschine im Wesentlichen senkrecht zur Oberfläche 110 des Straßenbaumaterials 50 angeordnet ist, beträgt der Verdrehwinkel α = arctan ((2,00 - 7d) / (h /cos γ_{F})) = arctan ((2,00 - 1,75) / (3,5 / cos 15)) = arctan 0,07 ≈ 3,95 °. Beim Erreichen des Messpunktes 103 beträgt der einzustellende Verdrehwinkel α folglich 0 °, da der Infrarottemperaturmesskopf 20 sich dann wieder in der bereits genannten Nullposition befindet. Die nun folgenden einzustellenden Verdrehwinkel berechnen sich analog zum Beispiel der Ausführungsform gemäß der Figuren 3 und 4, jedoch mit einer Montagehöhe h = 3,5 m. Demnach ist der erste, nach dem Messpunkt 103 folgende und in Richtung der rechten Außenkante 112 gerichtete, Verdrehwinkel β ≈3,95 °. Für den dann folgenden und im Abstand d = 0,25 m in Richtung der rechten Außenkante 112 liegenden Messpunkt 100 muss dann ein Verdrehwinkel von β ≈ 7,86 ° eingestellt werden. Der darauf folgende Verdrehwinkel β beträgt dann ≈ 11,69 °. Die weiteren einzustellenden Verdrehwinkel β des Infrarottemperaturmesskopfes 20 berechnen sich analog dazu.

Fig. 6 zeigt im Wesentlichen die am Straßenfertiger angeordnete Vorrichtung gemäß der Figur 5, wobei die Vorrichtung in Figur 6 um einen Montagewinkel γ_{S} im Bereich von ca. 15 ° in Scanrichtung des Infrarottemperaturmesskopfes 20 verdreht angeordnet ist. Dabei ist eine derartige Verdrehung üblicherweise montagebedingt, welche jedoch zur Erfassung der gesamten Einbaubreite B des neu aufgebrachten Straßenbelags 50 nicht zwingend erforderlich ist und auf das Arbeitsverhalten der Vorrichtung selbst bzw. auf den Infrarottemperaturmesskopf 20 keinen Einfluss hat.

Was die Ansteuerung des Motors 30, welcher den Infrarottemperaturmesskopf 20 quer zur Fahrtrichtung der Baumaschine verdreht, betrifft, so erfolgen die Berechnungen analog denen gemäß Figur 5, wobei der Montagewinkel γ_{S} mit in die Berechnung einfließt. Somit liegt auch hierbei der erste aufzunehmende Messpunkt 100 im Bereich der Außenkante 111 im Abstand B₁ von 2,00 m, ausgehend vom Messpunkt 103, welcher für den Infrarottemperaturmesskopf 20 die bereits genannte Nullposition bildet. Demnach wird für den Infrarottemperaturmesskopf 20 ein erster Verdrehwinkel α + γ_{S} = arctan (2,00 / (h /cos γ_{F})) + γ_{S} = arctan (2,00 / (3,5 / cos 15)) + 15 ≈ arctan 0,55 + 15 ≈ 43,9 ° eingestellt werden. Für den dann folgenden und im Abstand d = 0,25 m in Richtung der rechten Außenkante 112 liegenden Messpunkt 100 muss ein Verdrehwinkel von α + γ_{S} = arctan ((2,00 - d) / (h /cos γ_{F})) + γ_{S} = arctan ((2,00 - 0,25) / (3,5 / cos 15)) +15 ≈ arctan 0,48 + 15 ≈ 40,78 ° eingestellt werden. Der darauf folgende Verdrehwinkel α + γ_{S} beträgt dann arctan ((2,00 - 2d) / (h /cos γ_{F})) + γ_{S} = arctan ((2,00 - 0,50) / (3,5 / cos 15)) + 15 ≈ arctan 0,41 + 15 ≈ 37,49 °. Die weiteren einzustellenden Verdrehwinket α + γ_{S} berechnen sich analog dazu.

In Annäherung an den Messpunkt 103, an welchem der Infrarottemperaturmesskopf 20 in Fahrtrichtung der Baumaschine im Wesentlichen senkrecht zur Oberfläche 110 des Straßenbaumaterials 50 angeordnet ist, beträgt der Verdrehwinkel α + γ_{S} = arctan ((2,00 - 7d) / (h /cos γ_{F})) + γ_{S} = arctan ((2,00 - 1,75) / (3,5 / cos 15)) +15 ≈ arctan 0,07 ≈ 18,95 °. Beim Erreichen des Messpunktes 103 beträgt der einzustellende Verdrehwinkel α + γ_{S} folglich 15 °, da der Infrarottemperaturmesskopf 20 sich dann wieder in der bereits genannten Nullposition befindet. Die nun folgenden einzustellenden Verdrehwinkel berechnen sich ebenfalls analog zum Beispiel der Ausführungsform gemäß der Figur 5, jedoch auch unter Berücksichtigung des Montagewinkels γ_{S}. Demnach ist der erste, nach dem Messpunkt 103 folgende und in Richtung der rechten Außenkante 112 gerichtete, Verdrehwinkel β - γ_{S} ≈ -11,05 °. Für den dann folgenden und im Abstand d = 0,25 m in Richtung der rechten Außenkante 112 liegenden Messpunkt 100 muss dann ein Verdrehwinkel von β - γ_{S} ≈ -7,14 ° eingestellt werden. Der darauf folgende Verdrehwinkel β - γ_{S} beträgt dann ≈ -3,31 °.

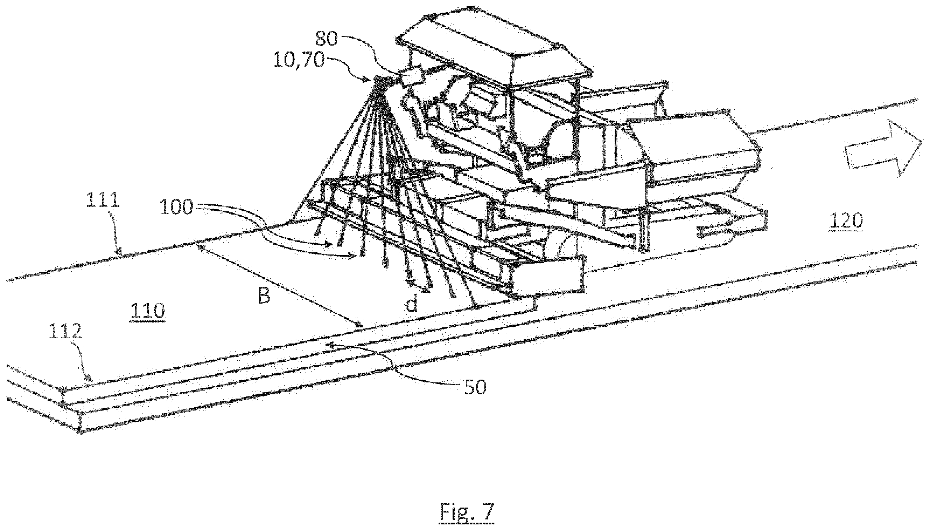

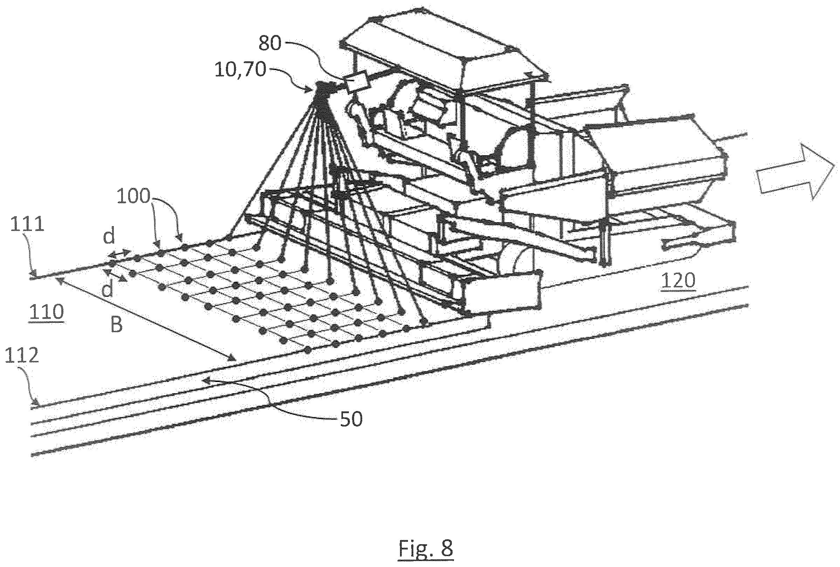

Der in Fig. 7 schematisch dargestellte Straßenfertiger weist an seinem hinteren Ende an der Position 10 die erfindungsgemäße Vorrichtung auf. Die Fahrtrichtung des Straßenfertigers ist durch einen Pfeil auf dem Untergrund 120 dargestellt. Im Bereich der erfindungsgemäßen Vorrichtung sind weiterhin ein Entfernungsmesser 70, beispielsweise ein Laserentfernungsmesser, und eine Wetterstation 80, welche beispielsweise die Windgeschwindigkeit und die Umgebungstemperatur im Bereich des Straßenfertigers ermittelt, angeordnet. Die erfindungsgemäße Vorrichtung misst die Temperatur der Oberfläche 110 des neu aufgebrachten Straßenbelags 50 über die Einbaubreite B, welcher durch die Außenkanten 111 und 112 seitlich, d. h. quer zur Fahrtrichtung des Straßenfertigers, begrenzt wird. Dabei werden die Messwerte an den schematisch dargestellten und im gleichmäßigen Abstand d angeordneten Messpunkten 100 quer zur Fahrtrichtung des Straßenfertigers aufgenommen. Bei einer Bewegung des Straßenfertigers in Fahrtrichtung führt die Abtastbewegung des Infrarottemperaturmesskopfes 20 zu Messpunkten 100 auf einer Messreihenline, welche in der Realität betrachtet schräg verläuft. In diesem Zusammenhang sei erwähnt, dass die Darstellung der Punkte in den Figuren 7 und 8 rein schematisch ist und lediglich dem Verständnis der Arbeitsweise der Vorrichtung dient.

Fig. 8 zeigt im Wesentlichen den Straßenfertiger aus Fig. 7, jedoch mit einem auf der Oberfläche 110 des neu aufgebrachten Straßenmaterials 50 und unmittelbar hinter der Asphaltbohle schematisch dargestellten Messpunkteraster. Die Messpunkte 100 weisen dabei sowohl quer zur Fahrtrichtung der Baumaschine als auch in Fahrtrichtung der Baumaschine einen gleichen Abstand d zueinander auf, sodass sich hinter der Asphaltbohle ein gleichbleibendes Messpunkteraster über die gesamte Einbaubreite B des neu aufgebrachten Straßenmaterials 50 ergibt.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Bezugszeichenliste

- 10: Montageposition der Vorrichtung
- 15: Gehäuse
- 16: Öffnung
- 20: Infrarottemperaturmesskopf
- 25: Infrarot-Strahlung
- 30: Motor
- 40: Ansteuerung
- 50: Straßenbaumaterial
- 50a: Untergrund
- 70: Entfernungsmesser
- 80: Wetterstation
- 100: Messpunkt
- 101, 102, 103: Messpunkt
- 110: Oberfläche
- 111, 112: Außenkanten
- 120: Untergrund
- d: Abstand zweier Messpunkte
- h: Montagehöhe
- A: Abstand
- α, β: Winkel
- γ_{S}, γ_{F}: Montagewinkel Vorrichtung (S = Scanrichtung; F = Fahrtrichtung)
- B: Einbaubreite
- B₁, B₂: Breite
- b: Bewegungsrichtung
- S₁, S₂: Infrarot-Strahlung an den Außenkanten

## Patentansprüche

1. Vorrichtung zur Bestimmung der Temperatur eines durch eine Baumaschine in einer Einbaubreite (B) aufgebrachten Straßenbaumaterials (50), mit:
einem Infrarottemperaturmesskopf (20),
einem Motor (30), und
einer Ansteuerung (40),
wobei der Infrarottemperaturmesskopf (20) durch den Motor (30) quer zur Fahrtrichtung der Baumaschine verdrehbar angeordnet ist und wirksam ist, um während einer Drehbewegung an mindestens zwei voneinander beabstandeten Messpunkten (100) Temperaturmesswerte der Oberfläche (110) des Straßenbaumaterials (50) aufzunehmen,
**dadurch gekennzeichnet, dass**
die Ansteuerung (40) wirksam ist, bei einer Montage der Vorrichtung an der Baumaschine im Bereich innerhalb der Einbaubreite (B) den Motor (30) basierend auf der Montageposition (10) der Vorrichtung an der Baumaschine derart anzusteuern, dass der Abstand der Messpunkte (100) auf der zu messenden Oberfläche (110) gleich bleibt,
wobei der Abstand (d) der Messpunkte (100) und/oder die Zeitdauer der Temperaturmessung an einem Messpunkt (100) einstellbar ist.

2. Vorrichtung nach Anspruch 1, **dadurch gekennzeichnet, dass** die Ansteuerung (40) wirksam ist, den Motor (30) ferner basierend auf den Montagewinkeln (γ_{S}, γ_{F}) der Vorrichtung an der Baumaschine anzusteuern.

3. Vorrichtung nach Anspruch 1 oder 2, **dadurch gekennzeichnet, dass** die Ansteuerung (40) wirksam ist, eine Bewegungsgeschwindigkeit des Infrarottemperaturmesskopfs (20) in Abhängigkeit von der Fahrgeschwindigkeit der Baumaschine zu ändern.

4. Vorrichtung nach einem der vorhergehenden Ansprüche, **dadurch gekennzeichnet, dass** die Ansteuerung (40) wirksam ist, die Bewegungsrichtung des Infrarottemperaturmesskopfs (20) zu wechseln, sobald die gemessene Temperatur an mindestens einem Messpunkt (100) einen vorbestimmten Mindestwert unterschreitet.

5. Vorrichtung nach Anspruch 4, **dadurch gekennzeichnet, dass** die Position, an der der Infrarottemperaturmesskopf (20) die Bewegungsrichtung wechselt, in der Ansteuerung (40) oder in einer an der Vorrichtung oder an der Baumaschine angeordneten Auswerteeinheit für eine Berechnung der Einbaubreite (B) des neu aufgebrachten Straßenbaumaterials (50) abgespeichert wird.

6. Vorrichtung nach einem der vorhergehenden Ansprüche, **dadurch gekennzeichnet, dass** die Vorrichtung einen berührungslosen Entfernungsmesser (70) umfasst, der wirksam ist, den Abstand (A) des Infrarottemperaturmesskopfes (20) zu dem Messpunkt (103) zu messen, an welchem der Infrarottemperaturmesskopf (20) im Wesentlichen senkrecht auf die Oberfläche (110) des Straßenbaumaterials (50) gerichtet ist.

7. Vorrichtung nach Anspruch 6, **dadurch gekennzeichnet, dass** der berührungslose Entfernungsmesser (70) mit der Ansteuerung (40) elektrisch verbunden ist.

8. Vorrichtung nach einem der vorhergehenden Ansprüche, **dadurch gekennzeichnet, dass** die Ansteuerung (40) mit einer an der Baumaschine angeordneten Wetterstation (80), welche Windgeschwindigkeit, Umgebungstemperatur, Luftfeuchte, Niederschlagsmenge und/oder andere Umgebungsparameter im Bereich der Baumaschine ermittelt, elektrisch verbindbar ist.

9. Vorrichtung nach einem der vorhergehenden Ansprüche, **dadurch gekennzeichnet, dass** der Motor (30) ein Schrittmotor, ein Servomotor, ein Gleichstrommotor oder ein Gleichstrommotor mit Getriebe ist.

10. Baumaschine mit mindestens einer Vorrichtung nach einem der vorhergehenden Ansprüche, wobei die Vorrichtung im hinteren Bereich und/oder im vorderen Bereich der Baumaschine angeordnet ist.

## Claims

1. A device for determining the temperature of a road building material (50) applied by a construction machine in a mounting width (B), comprising:
an infrared temperature measuring head (20),
a motor (30), and
a controller (40),
wherein the infrared temperature measuring head (20) is arranged to be twistable by the motor (30) transverse to the direction of travel of the construction machine and is effective to record temperature measuring values of the surface (110) of the road building material (50) during a rotational movement at at least two measuring points (100) spaced apart from one another,
**characterized in that** the controller (40) is effective to control, when fitting the device at the construction machine in the region within the mounting width (B), the motor (30) based on the fitting position (10) of the device at the construction machine such that the distance between measuring points (100) on the surface (110) to be measured remains equal
wherein the distance (d) of the measuring points (100) and/or the duration of the temperature measurement at a measuring point (100) is/are settable.

2. The device in accordance with claim 1, **characterized in that** the controller (40) is effective to control the motor (30) additionally based on the fitting angles (γ_{S}, γ_{F}) of the device at the construction machine.

3. The device in accordance with claim 1 or 2, **characterized in that** the controller (40) is effective to change a speed of movement of the infrared temperature measuring head (20) in dependence on the speed of travel of the construction machine.

4. The device in accordance with any one of the preceding claims, **characterized in that** the controller (40) is effective to change the direction of movement of the infrared temperature measuring head (20) as soon as the measured temperature falls below a predetermined minimum value at at least one measuring point (100).

5. The device in accordance with claim 4, **characterized in that** the position where the infrared temperature measuring head (20) changes its direction of movement is stored in the controller (40) or in an evaluating unit arranged at the device or at the construction machine for calculating the mounting width (B) of the newly applied road building material (50).

6. The device in accordance with any one of the preceding claims, **characterized in that** the device comprises a contactless distance measurer (70) which is effective to measure the distance (A) of the infrared temperature measuring head (20) to the measuring point (103) where the infrared temperature measuring head (20) is directed to the surface (110) of the road building material (50) essentially perpendicularly.

7. The device in accordance with claim 6, **characterized in that** the contactless distance measurer (70) is electrically connected to the controller (40).

8. The device in accordance with any one of the preceding claims, **characterized in that** the controller (40) is connectable electrically to a weather station (80) arranged at the construction machine which determines a wind speed, ambient temperature, air humidity, rainfall and/or another ambient parameter in the region of the construction machine.

9. The device in accordance with any one of the preceding claims, **characterized in that** the motor (30) is a stepper motor, a servomotor, a direct-current motor or a direct-current motor including a gear unit.

10. A construction machine comprising at least one device in accordance with any one of the preceding claims, wherein the device is arranged in the back region and/or in the front region of the construction machine.

## Revendications

1. Dispositif permettant de déterminer la température d'un matériau de voierie (50) appliqué sur une largeur de pose (B) par un engin de construction , avec:
une tête de mesure de température infrarouge (20),
un moteur (30), et
une commande (40),
dans lequel la tête de mesure de température infrarouge (20) est disposée par le moteur (30) de manière à pouvoir tourner transversalement par rapport au sens de déplacement de l'engin de construction et est active pour enregistrer pendant un mouvement de rotation à au moins deux points de mesure (100) distants l'un de l'autre les valeurs de mesure de température de la surface (110) du matériau de voierie (50),
**caractérisé par le fait que**
la commande (40) est active pour activer, lors d'un montage du dispositif sur l'engin de construction dans la largeur de pose (B), le moteur (30) sur base de la position de montage (10) du dispositif sur l'engin de construction de sorte que la distance entre les points de mesure (100) reste la même sur la surface à mesurer (110),
dans lequel la distance (d) entre les points de mesure (100) et/ou la durée de la mesure de température à un point de mesure (100) est réglable.

2. Dispositif selon la revendication 1, **caractérisé par le fait que** la commande (40) est active pour activer le moteur (30) par ailleurs sur base des angles de montage (y_{S}, y_{F}) du dispositif sur l'engin de construction.

3. Dispositif selon la revendication 1 ou 2, **caractérisé par le fait que** la commande (40) est active pour modifier une vitesse de déplacement de la tête de mesure de température infrarouge (20) en fonction de la vitesse de déplacement de l'engin de construction.

4. Dispositif selon l'une des revendications précédentes, **caractérisé par le fait que** la commande (40) est active pour changer le sens de déplacement de la tête de mesure de température infrarouge (20) dès que la température mesurée à au moins un point de mesure (100) descend au-dessous d'une valeur minimale prédéterminée.

5. Dispositif selon la revendication 4, **caractérisé par le fait que** la position à laquelle la tête de mesure de température infrarouge (20) change le sens de déplacement est mémorisée dans la commande (40) ou dans une unité d'évaluation disposée sur le dispositif ou sur l'engin de construction pour un calcul de la largeur de pose (B) du matériau de voierie (50) nouvellement appliqué.

6. Dispositif selon l'une des revendications précédentes, **caractérisé par le fait que** le dispositif comporte un télémètre sans contact (70) qui est actif pour mesurer la distance (A) entre la tête de mesure de température infrarouge (20) et le point de mesure (103) auquel la tête de mesure de température infrarouge (20) est dirigée sensiblement perpendiculairement à la surface (110) du matériau de voierie (50).

7. Dispositif selon la revendication 6, **caractérisé par le fait que** le télémètre sans contact (70) est connecté électriquement à la commande (40).

8. Dispositif selon l'une des revendications précédentes, **caractérisé par le fait que** la commande (40) peut être connectée électriquement à un poste météorologique (80) disposé sur l'engin de construction qui détermine la vitesse du vent, la température ambiante, l'humidité de l'air, la quantité de précipitation et/ou d'autres paramètres environnementaux à l'endroit de l'engin de construction.

9. Dispositif selon l'une des revendications précédentes, **caractérisé par le fait que** le moteur (30) est un moteur pas à pas, un servomoteur, un moteur à courant continu ou un moteur à courant continu à engrenage.

10. Engin de construction avec au moins un dispositif selon l'une des revendications précédentes, dans lequel le dispositif est disposé dans la zone arrière et/ou dans la zone avant de l'engin de construction.
